# EUROPEAN PATENT APPLICATION

(11) **EP 3 135 272 A1**
(43) Date of publication of application: **01.03.2017**
(21) Application number: 16186228.9
(22) Date of filing: 30.08.2016
(51) Int. Cl.: A61K 8/34, A61Q 11/00, A61K 8/81, A61K 8/86, A61K 9/00, A61K 9/20

(54) **COMPOSITIONS FOR MOUTH WASH IN THE FORM OF TABLETS**

(30) Priority: 31.08.2015 EP 15183155
(71) Applicant: BASF S.A., 04794-000 Sao Paulo (BR)
(72) Inventor: JULIAN JUNIOR, Luis, 12244-396 São José dos Campos (BR); ITO, Fabio Akira, 04055-110 SÃO PAULO (BR)
(74) Representative: Reitstötter Kinzebach

(57) **Abstract**

The present invention relates to formulations in the form of agglomerates for the production of tablets comprising sugars or sugar alcohols, crosslinked polyvinylpyrrolidone and water-soluble polymers, for the production of administration forms for use in mouth and throat. Compositions for use in mouth and throat which not only leave behind a pleasant mouthfeel and are mechanically very stable, but also make possible simple usage and dosage and can also be administered without additional use of liquid are described herein.

## Description

The present invention relates to formulations in the form of agglomerates for the production of tablets comprising sugars or sugar alcohols, crosslinked polyvinylpyrrolidone and water-soluble polymers, for the production of administration forms for use in mouth and throat.

Tablets which disintegrate rapidly and/or dissolve rapidly in the mouth are gaining ever increasing importance for the oral application of medicaments. Such tablets must disintegrate in the oral cavity within a short time, optimally within 30 seconds, must have a pleasant taste and must not leave behind a sandy feeling. In addition, they should be simple to produce, with direct tableting offering considerable advantages over moist granulation, and have a high mechanical strength so that they withstand the packagings procedure, transport and also being pressed out of the packages undamaged. However, also chewing and sucking tablets which likewise disperse in the mouth are gaining increasing significance.

Such administration forms are also especially of interest for use for care of the mouth and throat, such as, for example, preventing mouth odor or caries, for tooth bleaching or for disinfecting the gums and throat. In the form of tablets, active compounds may be dosed more exactly for said applications compared with liquid or pasty formulations.

Rapidly disintegrating tablets frequently comprise sugar and sugar alcohols, effervescent systems, microcrystalline cellulose and other water-insoluble fillers, such as calcium hydrogenphosphate, cellulose derivatives, cornstarch or polypeptides. In addition, use is made of water-soluble polymers, conventional disintegrants (crosslinked PVP, sodium and calcium salts of crosslinked carboxymethyl cellulose, sodium salt of carboxymethyl starch, low-substituted hydroxypropyl cellulose (L-HPC)) and essentially inorganic water-insoluble components (silicas, silicates, inorganic pigments). In addition, the tablets can also comprise surfactants.

WO 2003/051338 describes a directly tabletable and easily pressable aid formulation which comprises mannitol and sorbitol. Firstly, by dissolving mannitol and sorbitol in water and subsequently spray drying (conventional spray drying and SBD process), an aid premix is produced. Mannitol can additionally be added to this coprocessed mixture. Tablets which additionally comprise disintegrant, release agent, pigment and an active compound are said to disintegrate in the oral cavity in the course of 60 seconds.

US 2002/0071864 A1 describes a tablet which disintegrates in the oral cavity in the course of 60 seconds and is principally formulated from a physical mixture of spray-dried mannitol and a coarse-grained crosslinked polyvinylpyrrolidone and also a limited selection of active compounds. These tablets have a breaking strength of approximately 40N and produce an unpleasant, sandy mouthfeel.

According to US 6,696,085 B2, a methacrylic acid copolymer type C should be used as disintegrating composition. The methacrylic acid copolymer type C is a gastric juice-resistant polymer which is insoluble in the acidic pH range, but water-soluble in the pH range of 7 as exists in the oral cavity. The tablets, in addition to a low breaking strength (< 20N), have a high friability (> 7%) and have a high content in the range of 15% by weight of a coarse-grained disintegrant. They consequently have a low mechanical strength and, owing to the high content of coarse-grained disintegrant, produce an unpleasant sandy mouthfeel.

EP 0839526 A2 describes a pharmaceutical administration form comprising an active compound, erythritol, crystalline cellulose and a disintegrant. In addition, mannitol is incorporated and, as disintegrant, use is made of crosslinked polyvinylpyrrolidone, such that a physical mixture is formed. The tablets are said to disintegrate in the oral cavity in the course of 60 seconds.

In the application JP 2004-265216, a tablet disintegrating in the mouth in the course of 60 seconds is described which comprises an active compound, a water-soluble polyvinyl alcohol-polyethylene glycol copolymer, sugar/sugar alcohol (mannitol) and disintegrant.

Deodorizing and caries-inhibiting tablets are known, for example, from US 2,894,876 and US 3,556,811. US 4,041,149 describes formulations in the form of mouthwash, toothpaste or dental cream which, as filler, comprise a sugar alcohol. Polyvinyl acetate-comprising chewing gums are also described.

Tablets for oral care based on sugar alcohols which comprise an active combination of zinc salts and ionone compounds are disclosed by DE 37 06 142 A.

The matrix components based on sugar alcohols, disintegrants and insoluble polymers are generally known for pharmaceutical applications from WO 2007/071581.

WO 2009/037114 A1 reads on a mouthwash composition which can be a tablet rapidly disintegrating in the mouth. This tablet may comprise in its matrix a water-soluble ethylene oxide-propylene oxide block copolymer.

It was an object of the present invention to find improved compositions for use in mouth and throat which not only leave behind a pleasant mouthfeel and are mechanically very stable, but also make possible simple usage and dosage, and also can be administered without additional use of liquid.

Additionally, certain active compounds having disinfectant activity such as triclosan present formulation diificulties because they are practically insoluble in water. Even when they are formulated n tablets, this disadvantage may jeopardize their antimicrobial activity when the tablet disintegrates in the mouth. It was thus another object of the present invention to increase the solubility as well as the antimicrobial activity of active compounds having desinfactant activity.

Accordingly, compositions have been found for use in mouth and throat which, in addition to at least one active compound, comprise as matrix component a mixture of
a) 60-98% by weight of at least one sugar or sugar alcohol or mixtures thereof,
b) 1-25% by weight of a disintegrant,
c) 1-15% by weight of water-insoluble polymers,
d) 1-15% by weight of water-soluble polymers, and
e) 0-15% by weight of further aids,
wherein the sum of components a) to e) is 100% by weight.

In addition, administration forms, in particular tablets which disintegrate rapidly in the mouth, comprising such preparations have been found. The rapidly disintegrating tablets disintegrate in the mouth or in an aqueous environment in the course of 40 seconds, preferably in the course of 30 seconds, particularly preferably in the course of 20 seconds. Likewise, sucking and chewing tablets obtainable from such compositions have been found. Likewise, the use of such compositions in the form of sachets has been found.

In addition, it has surprisingly been found that oxidizing and reducing, and also disinfecting active compounds can be formulated in such rapidly disintegrating administration forms with the help of water-soluble polymers (components d) while retaining the stability of the formulation and of the active compounds.

Active compounds for the care of mouth and throat according to the invention are active compounds for oral and dental hygiene, for the treatment of mouth odor, caries, tooth plaques, tooth discolorations and fine disinfection of the gums and of the mouth and throat mucosa.

The preparations comprise, as component a), 60 to 98% by weight, preferably 70 to 95% by weight, particularly preferably 75 to 93% by weight, of a sugar, sugar alcohol or mixtures thereof. Suitable sugars or sugar alcohols are trehalose, mannitol, erythritol, isomalt, maltitol, lactitol, xylitol, sorbitol. The sugar or sugar alcohol components are preferably finely divided, having median particle sizes of 5 to 100 µm. If desired, the particle sizes can be set by milling. Preferably, use is made of mannitol, erythritol or mixtures thereof.

As component b), use is made of disintegrants in amounts of 1 to 25% by weight, preferably 2 to 15% by weight, particularly preferably 3 to 10% by weight. Such disintegrants are water-insoluble, but not film-forming. Suitable disintegrants are crosslinked polyvinylpyrrolidone (crospovidone), croscarmellose, a crosslinked carboxymethylcellulose, wherein croscarmellose, according to the invention, also means its sodium and calcium salts. Also suitable is sodium carboxymethyl starch. Likewise suitable is L-hydroxypropylcellulose, preferably having 5 to 16% hydroxypropoxy groups, as described in USP/NF 2005. Preferably, use is made of crospovidone.

As component c), use is made of water-insoluble polymers in amounts of 1 to 15% by weight, preferably 1 to 10% by weight. These are polymers. Preference is given to polymers which are insoluble in the pH range from 1 to 14, that is have a pH-independent water insolubility at any pH. In addition, however, polymers are also suitable which are water insoluble at any pH in the pH range from 6 to 14.

The polymers shall be film-forming polymers. Film-forming, in this context, means that the polymers in aqueous dispersion have a minimum film-forming temperature of -20 to +150°C, preferably 0 to 100°C.

Suitable polymers are poly(vinyl acetate), ethylcellulose, methyl methacrylate-ethyl acrylate copolymers, ethyl acrylate-methyl methacrylate-trimethylammonium methyl methacrylate terpolymers. Butyl methacrylate-methyl methacrylate-dimethylaminoethyl methacrylate-terpolymers.
The acrylate-methacrylate copolymers are described in more detail in the European Pharmacopoeia as Polyacrylate Dispersion 30%, in the USP as Ammonio Methacrylate Copolymer and in JPE as Aminoalkyl-Methacrylate Copolymer E.
As preferred component c), use is made of poly(vinyl acetate). This is can be used as aqueous dispersion having solids contents of 10 to 45% by weight. Preference, in addition, is given to poly(vinyl acetate) having a molecular weight between 100 000 and 1 000 000 daltons, particularly preferably between 200 000 and 800 000 daltons.

In addition, the formulation comprises, as component d), water-soluble polymers in amounts of 1 to 15% by weight, preferably 5 to 10% by weight. Suitable water-soluble polymers are ethylene oxide (EO)-propylene oxide(PO) block copolymers arranged in a triblock structure EOₓ-PO_{y}-EOₓ. Preferably component d) has an average molecular range lying in the range of from 9,840g/mol to 14,600g/mol, x lies in the range of from 95 to 105 and y lies in the range of from 54 to 60.

If desired, flavor and appearance of the tablets obtained from the formulations can be further improved by addition of pharmaceutically conventional aids (component e)) in amounts of 0 to 15% by weight, for example such as acidulates, buffer substances, sweeteners, flavorings, flavor enhancers and dyes.

The following substances are particularly suitable in this case:
Suitable acidulants are, for example, citric acid, tartaric acid, ascorbic acid and sodium dihydrogenphosphate.

Suitable sweeteners are, for example, cyclamate, saccharin-Na, aspartame and neohesperidin.

Suitable flavorings are, for example, fruit flavorings, vanilla flavoring, cocoa flavoring, glutamate, menthol.

Suitable dyes are: riboflavin, curcumin, beta-carotene, water-soluble dyes as are used for coloring foods, and also finely divided color lakes.

By addition of thickeners such as high-molecular-weight polysaccharides, the mouthfeel can be additionally improved by increasing the softness and the volume feel.

In addition, as component e), use can also be made of surfactants. Suitable surfactants are, for example, sodium lauryl sulfate, dioctyl sulfosuccinate, alkoxylated sorbitan esters such as polysorbate 80, polyalkoxylated derivatives of castor oil or hydrogenated castor oil, for example Cremophor® RH 40, alkoxylated fatty acids, alkoxylated hydroxy fatty acids, alkoxylated fatty alcohols, alkali metal salts of fatty acids, and lecithins. In addition, sodium stearyl fumarate is suitable.

In addition, for further improvement of the disintegration, finely divided pigments can also be added because they increase the internal interfaces and water can thereby penetrate more rapidly into the tablet. These pigments, such as iron oxides, titanium dioxide, colloidal or precipitated silica, calcium carbonates, calcium phosphates, must of course be very finely divided, otherwise again a grainy taste results.

The mixture of components a) to e) is preferably used in the form of agglomerates.

The agglomerates can be produced by build-up agglomeration in mixers, fluidized-bed apparatuses or spray towers. In this process solid starting materials and granulation liquid are first mixed with one another and the moist mixed material is subsequently dried. According to the present invention, the granulation liquid used is an aqueous dispersion of component c), the water-insoluble polymer.

In the case of agglomeration in the fluidized bed, an aqueous dispersion of the water-insoluble polymer is sprayed onto a fluidized mixture of sugar or sugar alcohol and disintegrant, as a result of which the fine particles agglomerate. The feed air temperatures are 30 to 100°C, the exhaust air temperatures 20 to 70°C.

In the case of production in spray towers, preferably fluidized spray drying (FSD) or spray bed drying (SBD) technology is used. In this case a solution of the sugar or sugar alcohol in water is first spray dried, and in the lower part of the spray dryer or in an attached fluidized bed the disintegrant is added and an aqueous dispersion of the water-insoluble polymer is injected, as a result of which the particles agglomerate. Fine particles can in addition be blown again upstream of the spraying nozzle of the sugar or sugar alcohol solution and additionally agglomerated. In the spraying tower, FSD or SBD, a process procedure is also possible starting from the crystalline form of the sugar or sugar alcohol. In this case the crystalline sugar or sugar alcohol is added at the top of the spraying tower or into the fine material recycling stream. By spraying an aqueous dispersion of the water-insoluble polymer, this crystalline solid is agglomerated in the tower.

For the agglomeration process, it may be advantageous to run a multistage spraying process. At the start the spraying rate is kept low in order to prevent overwetting of the product charge and thus prevent it sticking together. With increasing process time, the spraying rate can be increased and thereby the agglomeration tendency increased. It is likewise possible to adapt the feed air rate and/or temperature in a corresponding manner during the process. Particularly during the drying phase it is advantageous to reduce the feed air rate and thereby avoid abrasion of the agglomerates due to high mechanical stress.

The fineness of the spray droplet of the binder solution or dispersion (which can be set via the atomizing gas pressure), the nozzle geometry and distance of the nozzle from the product bed can be considered as further adaptation parameters for the agglomerate size. The finer and more uniform the spraying is, the finer and more uniform are the resulting agglomerates. The further the nozzle is from the product bed, the worse is the agglomeration behavior.

In addition, the agglomerates can also proceed in a mixer via a continuous mixing aggregation. Such a continuously conducted form of mixing aggregation is what is termed "Schugi granulation". In this case, in a continuous vertically arranged highspeed mixer, solid starting materials and the granulation liquid comprising the water-insoluble polymer are intensively mixed with one another (see also M. Bohnet, "Mechanische Verfahrenstechnik" [Mechanical Process Engineering], Wiley VCH Verlag, Weinheim 2004, pp. 198 ff.).

According to a particular embodiment, the disintegrant is suspended in the aqueous dispersion of the water-insoluble polymer.
The resultant agglomerates have a median particle size of 70-600 µm, preferably 120-500 µm, and particularly preferably 140-400 µm.
The water-insoluble film-forming polymer acts in this case as agglomeration agent in order to agglomerate the fine sugars or sugar alcohol crystals and the particles of the disintegrant.

The formulations according to the invention can be used advantageously for producing compositions for treatment of mouth and throat.

The compositions for treatment of mouth and throat comprise, in addition to the mixture acting as matrix former, active compounds especially for the care and treatment of mouth and throat (oral care). According to the invention this is taken to mean including oral and dental hygiene, combating halitosis (mouth odor), caries, tooth plaques, tooth discolorations and fine disinfection for combating microbially caused inflammatory changes of the mouth and throat mucosa.

Suitable active compounds can be:
complexes of iodophores with vinylpyrrolidone polymers such as polyvinylpyrrolidone, wherein the vinylpyrrolidone polymers can be either water-soluble polyvinylpyrrolidones or water-insoluble crosslinked polyvinylpyrrolidones (also called crospovidones). Preference is given to polyvinylpyrrolidone-iodine. The production of such compounds which are also commercially available is known per se to those skilled in the art. Surprisingly it has been found that such reactive active compounds can be formulated so as to be stable in the claimed administration forms.

Iodophores are compounds which can release iodine. Examples are complexes of elemental iodine with hydrogen iodide and polyvinylpyrrolidone (polyvinylpyrrolidone-iodine, also termed povidone-iodine or - if water-insoluble crosslinked polyvinylpyrrolidone is used - crospovidone-iodine; a generally useful abbreviation is PVP-iodine).

Preference is given to the use of a PVP-iodine having relatively small particles such as a micronized PVP-iodine (for example PVP-iodine 30/06 M10 from BASF), since the color of PVP-iodine becomes lighter with decreasing particle size. The color of a PVP-iodine 30/06M10-comprising composition is thereby lighter than that of a composition which comprises the non-micronized PVP-iodine 30/06. Therefore, for example, dyeing of the composition using further coloring substances can be achieved more readily. Also - if dyeing of the composition is omitted - an orange coloration of the composition when the micronized PVP-iodine is used is a color which is more preferred by users than a brownish-red coloration of the composition when the non-micronized PVP-iodine is used.

Surprisingly it has also been found that such complexes with iodophores can also be produced in situ and used for the compositions according to the invention, as a result of which the orange to brown coloration which is an inherent property of the PVP-iodine complexes may be avoided and thus colorless or virtually colorless compositions can be prepared without the desired disinfectant activity of the PVP-iodine complexes having to be relinquished:
The complexes with iodine can be prepared in situ on contact of the composition with water by redox reaction of iodides with iodates. Preference is given to the use of sodium salts of the iodide and sodium salts of the iodate. The molar ratio of iodide to iodate in this case is 10:1 to 6:1, preferably 9:1 to 7:1, and particularly preferably 8.1:1 to 7.9:1.
The iodine salts are used in this case preferably in the anhydrous state or having very low amounts of residual water which can be present, for instance, in the form of bound water of crystallization. The amounts of water are at most 5 percent by weight based on the total mass of the sodium iodide and the sodium iodate, preferably at most 2% by weight, and very particularly preferably less than 1 percent by weight, in particular use is made of such salts of iodine which comprise up to 0.2 percent by weight of water or no water at all.

The reaction of iodides with iodates is known per se to those skilled in the art. Likewise known is the complexation of triiodide to polyvinylpyrrolidone:
in the reaction of iodides with iodates, in a rapid redox reaction elemental iodine is formed which, with an excess of iodides, participates in a complex compound to form the triiodide complex ion. This triiodide ion, together with polyvinylpyrrolidone and water-insoluble crosslinked polyvinylpyrrolidone, in particular in a slightly or strongly acidic environment, forms a complex which corresponds to the commercially available polyvinylpyrrolidone-iodine or crospovidone-iodine products. Preferably, such compounds are customarily formed from hydrogen iodide and elemental iodine, wherein the hydrogen iodide is formed in situ from the reaction of a proton-releasing reducing agent with elemental iodine. The molar ratios of reducing agent and iodine are customarily selected in this case in such a manner that after the reaction 1 mol of hydrogen iodide is present per mole of elemental iodine, so that 1 mol of hydrogen triiodide can form.

Further preferred active compounds are:
complexes of vinylpyrrolidone polymers such as polyvinylpyrrolidone, wherein the vinylpyrrolidone polymers can be not only water-soluble polyvinylpyrrolidones but also water-insoluble crosslinked polyvinylpyrrolidones:
   polyvinylpyrrolidone-hydrogen peroxide and/or water-insoluble crosslinked polyvinylpyrrolidone-hydrogen peroxide (crospovidone-hydrogen peroxide) may be mentioned as examples. Preference is given to water-soluble polyvinylpyrrolidonehydrogen peroxide and crospovidone-hydrogen peroxide, very particularly preferably water-soluble polyvinylpyrrolidone-hydrogen peroxide. The production of such complexes which are also commercially available is known per se to those skilled in the art. In such complexes, the amount of hydrogen peroxide can vary. For the present invention preference is given to 5 to 50 percent by weight, based on the PVP; particular preference is given to 15 to 35 percent by weight of hydrogen peroxide.

In addition, preferred active compounds are halogenated diphenyl ethers such as, for example, 2,4,4'-trichloro-2'-hydroxydiphenyl ether (triclosan).

In addition, other active compounds are suitable which can be used individually or as further components together with the abovementioned active compounds: zinc salts such as the fluorides, chlorides, iodides, gluconates, phenolsulfonates, copper salts such as the gluconates, inorganic fluorine salts of sodium, potassium, ammonium, calcium, copper(I), barium, zinc, in addition the fluorosilicates or fluorozirconates of sodium, potassium and ammonium, mono- or difluorophosphates of sodium or aluminum, fluorinated sodium calcium pyrophosphate.

In addition, suitable active compounds are the following organic compounds:
phenolic compounds such as eucalyptol, thymol, methyl salicylate, menthol, chlorothymol, phenol, halogenated and other derivatives of phenol such as methoxy-4-(2-propenyl)phenol (eugenol), alkyl-p-chlorophenols, alkyl-p-bromophenols, alkyl-o-bromophenols and the like; resorcinol and derivatives thereof; bisphenolic compounds;
halogenated salicylanilides such as, for example, 3,5-dibromo-3'-trifluoromethylsalicylanilide (fluorophen); esters of benzoic acid such as, for example, methyl p-hydroxybenzoate; halogenated carbanilides.

Such suitable organic compounds are listed, for example, in DE 695 23 513 T2 which is explicitly incorporated herein by reference.

Cetylpyridinium chloride, quaternary ammonium compounds such as, for example, didecyldimethylammonium chloride or benzalkonium chloride.

The amount of the active components used in a formulation varies depending on active compounds between 0.01 % and 25%, usually preferably in a range from 0.1 % to 15%.

For production of the compositions according to the invention, active compounds and matrix formers and preferably lubricants are mixed with one another, and an additional granulation step can also be carried out.

For the preparation of tablets, the conventional methods can be used, and direct tableting and roller compacting offer particular advantages. Owing to the particular properties of the formulations according to the invention, generally only active compound, formulation according to the invention, and a lubricating agent are required. The tablet formula is therefore very simple, very reproducible and the process can readily be validated.

It is also possible to produce two-layer or multilayer tablets, in which case different active compounds can be incorporated in the different layers.

Should the active compounds which are selected react with one another or initiate or accelerate the decomposition, this can be prevented by separating the active compounds from one another. This can be performed by producing two-layer or multilayer tablets, by coating one or more active compounds in the form of, for example, coated microparticles, by complexing one of the active compounds, for example, to polymers, or other suitable methods which are known to those skilled in the art.

When iodide and iodate salts are used, for example one layer of the multilayer tablets is produced with the iodide salt, the other with the iodate salt. As a result, there is spatial separation of the salts, in such a manner that a premature reaction and thereby the start of undesirable brown-orange coloration are avoided.
Likewise, for example, the iodine salts can be used as coated microparticles, in such a manner that contact of the iodide with the iodate can only proceed after dissolution of the coating.

Coatings which are preferably used are coatings which rapidly dissolve in polar, and in particular aqueous, media such as water or saliva. What are called aqueous media in the context of this invention are all mixtures of water with suitable solvents such as, for example, suitable alcohols such as ethanol or isopropanol. Also, the saliva which is naturally present in the mouth is such an aqueous medium.
Such coatings are known to those skilled in the art. Examples which may be mentioned are vinyl alcohol/polyethylene glycol graft copolymers (Kollicoat IR, BASF), hydroxypropylmethylcelluloses (HPMC) or poly(vinyl alcohol)-based coatings.

Preferably, such coating materials are selected and the film thickness of the coating is selected in such a manner that the film dissolves within a few minutes, preferably in less than 60 seconds, and very particularly preferably in less than 30 seconds, in which case the resultant iodine salts are brought into contact with the aqueous medium and thus a reaction of the iodide and iodate salts is enabled. Polymers of the Kollicoat IR group are particularly suitable as coatings.
If such iodine salts are used as a combination of, preferably, sodium iodide with, preferably, sodium iodate, the iodine salts are preferably processed in this case in a water-reduced atmosphere of less than 20% relative humidity, preferably less than 15% relative humidity, and very particularly preferably less than 10% relative humidity, in order to prevent premature reaction of the iodides with the iodates, or at least substantially avoid it. Conditions of 15% relative humidity and less are customarily used for producing moisture-sensitive effervescent tablets and formulations and are therefore known to those skilled in the art.

Therefore, in the case of multilayer tablets, coated microparticles or in the case of production under reduced humidity, in each case premature reaction and thereby the formation of the typical orange to brown coloration of the PVP-iodine complexes is avoided. In addition, as also in the case of the other active compounds according to the invention, reaction with other components of the formulation is avoided or at least greatly reduced.

This premature reaction is generally promoted by the presence of relatively large amounts of water, for example in the form of humidity. Therefore the production of such compositions proceeds preferably under reduced relative humidity. Preference is likewise given to the use of coated microparticles of the individual iodine salts.

Very particular preference is given to the use of active compounds which have been predried to the lowest possible amounts of water. These amounts of water are less than those which are conventionally present in the individual commercially available active compounds: the amounts of water are preferably less than 50 percent, particularly preferably less than 80 percent, very particularly preferably less than 95 percent and particularly preferably less than 99 percent of the amounts of water conventionally present in commercial active compounds.
The content of water in the active compounds which are used is therefore preferably up to 5 percent by weight, based on the total mass of the active compound, particularly preferably up to 2, very particularly preferably up to 0.5, and particularly preferably up to 0.2 percent by weight of water. Particular preference is given to the use of anhydrous salts, where the technique makes such an anhydrous state achievable.

In addition, the compositions according to the invention have extremely good flow properties and moldabilities which lead to very mechanically stable tablets. The breaking strength of the tablets produced using the pharmaceutical formulations according to the invention is > 50 N. Frequently, the breaking strengths are above 80 N, even with the use of active compounds which are difficult to mold. The friabilities are < 0.2%. Damage during conventional tablet handling therefore does not occur.

The dissolution rate can also be controlled via the molding pressure. If the intention is to produce rapidly disintegrating tablets, relatively low to medium molding pressures will be selected in the range from 3 to 10 kN. If the intention is to produce sucking tablets or chewing tablets, higher molding pressures in the range from 8 to 15 kN are advisable.

The formulations according to the invention are therefore very stable during storage and retain their attractive appearance.

Formulation example of mouthwash tablet according to the invention

Final weight of the tablet 430,0 mg

| Formulation | mg/comprimido | wt % |
|---|---|---|
| Ludiflash | 326,6 | 75,95 |
| Triclosan | 4,5 | 1,05 |
| Kollidon® CL-SF | 34,4 | 8,00 |
| Kolliphor P 407 Micro | 38,7 | 9,00 |
| Aroma Laranja | 4,3 | 1,00 |
| Mentol | 17,2 | 4,00 |
| Pruv | 4,3 | 1,00 |
| Total | 430,0 | 100,0 |

## Claims

1. A composition for use in mouth and throat which, in addition to at least one active compound having disinfectant activity, comprises as matrix component a mixture of
a) 60-98% by weight of at least one sugar or sugar alcohol or mixtures thereof,
b) 1-25% by weight of a disintegrant,
c) 1-15% by weight of water-insoluble polymers,
d) 1-15% by weight of water-soluble polymers, and
e) 0-15% by weight of further pharmaceutically conventional aids,
wherein the sum of components a) to e) is 100% by weight.

2. The composition according to claim 1, comprising, as sugar alcohol a), mannitol.

3. The composition according to claim 1 or 2, comprising, as component b), a polymer selected from the group consisting of crosslinked polyvinylpyrrolidone (crospovidone), croscarmellose, sodium and calcium salts of croscarmellose, sodium carboxymethyl starch and L-hydroxypropyl cellulose.

4. The composition according to one of claims 1 to 3, comprising, as component b), crospovidone.

5. The composition according to one of claims 1 to 4, comprising, as component c), polyvinyl acetate, ethyl cellulose, copolymers of (meth)acrylic acid or mixtures thereof.

6. The composition according to one of claims 1 to 5, comprising, as component d), ethylene oxide-propylene oxide block copolymers arranged in a triblock structure EOₓ-PO_{y}-EOₓ.

7. The composition according to claim 6, wherein component d) has an average molecular range lying in the range of from 9,840g/mol to 14,600g/mol , x lies in the range of from 95 to 105 and y lies in the range of from 54 to 60.

8. The composition according to one of claims 1 to 7, wherein component d) is present in the matrix in an amount of 5 to 15% by weight.

9. The composition according to one of claims 1 to 8, comprising, as component e), pharmaceutically conventional aids such as acidulates, buffer substances, sweeteners, flavorings, flavor enhancers and dyes.

10. The composition according to one of claims 1 to 9, comprising, as active compound, a mixture or a complex of iodophores with polyvinylpyrrolidone, wherein the polyvinylpyrrolidone can be water-solubly or water-insolubly crosslinked.

11. The composition according to one of claims 1 to 9, comprising triclosan and/or sodium fluoride and/or sodium monofluorphosphate as active compound.

12. The composition according to one of claims 1 to 11 in the form of a tablet or pastille obtained by compaction.

13. The composition according to one of claims 1 to 12 comprising one or more active compounds in amounts of 0.01 to 25% by weight, based on the total weight of the composition.

14. The use of compositions according to one of claims 1 to 13 for the treatment of mouth odor, caries, tooth plaque, tooth discolorations and microbially caused diseases of the gums and of the mouth and throat mucosa.
